# EUROPÄISCHE PATENTSCHRIFT

(11) **EP 2 595 790 B1**
(45) Veröffentlichungstag und Bekanntmachung des Hinweises auf die Patenterteilung: **01.06.2016**
(21) Anmeldenummer: 11764083.9
(22) Anmeldetag: 18.07.2011
(51) Int. Cl.: B29C 49/06, B29C 49/12, B29C 49/16, B29C 49/36, B29C 49/42, B29C 49/46, B29C 49/64, B65B 55/10, A61L 2/20, A61L 2/16, B29K 23/00, B29K 67/00

(54) **VERFAHREN UND VORRICHTUNG ZUM STERILISIEREN VON VORFORMLINGEN**
METHOD AND DEVICE FOR STERILIZING PREFORMS
PROCÉDÉ ET DISPOSITIF POUR LA STÉRILISATION DE PRÉFORMES

(30) Priorität: 22.07.2010 DE 102010032336
(43) Veröffentlichungstag der Anmeldung: 29.05.2013
(73) Patentinhaber: KHS Corpoplast GmbH, 22145 Hamburg (DE); KHS GmbH, 44143 Dortmund (DE)
(72) Erfinder: HEROLD, Thomas, 22926 Ahrensburg (DE); RIEGER, Harald, 22303 Hamburg (DE); KLATT, Dieter, 22147 Hamburg (DE); HAESENDONCKX, Frank, 22395 Hamburg (DE)
(74) Vertreter: Hausfeld, Norbert
(86) Internationale Anmeldenummer: PCT/DE2011/001486
(87) Internationale Veröffentlichungsnummer: WO 2012/010166

(56) Entgegenhaltungen:
- EP-A1- 1 941 913
- EP-A1- 2 295 324
- DE-A1-102007 050 582
- FR-A1- 2 815 542

## Beschreibung

Die Erfindung betrifft ein Verfahren zum Sterilisieren von Vorformlingen aus einem thermoplastischen Material, die zur Herstellung von blasgeformten Behältern vorgesehen sind sowie bei dem ein Sterilisationsmittel in den Bereich des Vorformlings zugeführt wird, sowie bei dem die Vorformlinge vor ihrer Blasverformung erwärmt werden.

Darüber hinaus betrifft die Erfindung eine Vorrichtung zum Blasformen von mindestens bereichsweise sterilen Behältern durch Verformung eines Vorformlings, die eine Zuführeinrichtung zur Beaufschlagung mindestens eines Teiles des Vorformlings mit einem Sterilisationsmittel aufweist.

Schließlich betrifft die Erfindung eine Vorrichtung zum Sterilisieren von Vorformlingen aus einem thermoplastischen Material, die zur Herstellung von blasgeformten Behältern vorgesehen sind. Ebenfalls ist von der Erfindung eine Vorrichtung zur Herstellung von mindestens bereichsweise sterilen Behältern aus einem thermoplastischen Material umfaßt.

Die Erfindung betrifft auch eine Vorrichtung zur Blasformung von Behältern, die mindestens eine auf einer Tragstruktur angeordnete Blasstation zur Umformung von thermoplastischen Vorformlingen in die Behälter aufweist.

Eine Herstellung von sterilen blasgeformten Behältern erfolgt typischerweise derart, daß diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Bei einer Behälterformung durch Blasdruckeinwirkung werden Vorformlinge aus einem thermoplastischen Material, beispielsweise Vorformlinge aus PET (Polyethylenterephtalat), innerhalb einer Blasmaschine unterschiedlichen Bearbeitungsstationen zugeführt. Typischerweise weist eine derartige Blasmaschine eine Heizeinrichtung sowie eine Blaseinrichtung auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling eingeleitet wird. Der verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE -OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die Verwendung von Greifzangen zur Handhabung von Vorformlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von Übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgußverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgußtechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Hinsichtlich der Sterilisierung von Vorformlingen sind aus dem Stand der Technik bereits unterschiedliche Verfahren und Vorrichtungen bekannt, die jedoch alle verfahrensspezifische Nachteile aufweisen, die einer zuverlässigen Sterilisierung der Vorformlinge bei gleichzeitig hohen Durchsatzraten entgegenstehen.

In der EP-A 1 086 019 wird beispielsweise die Sterilisierung von heißen Vorformlingen mit einem heißen gasförmigen Sterilisationsmittel beschrieben. Es werden hintereinander angeordnete separate Behandlungsstationen verwendet, nämlich ein erstes Heizmodul, ein Sterilisiermodul sowie ein zweites Heizmodul. Nachteilig ist hierbei das Temperaturverhalten des Vorformlings während des Sterilisiervorganges sowie das unkontrollierte Austreten des Sterilisationsmittels aus dem Vorformling innerhalb der Heizung.

In der EP-A 1 896 245 wird ein Verfahren beschrieben, bei dem vor der Heizung ein gasförmiges Sterilisationsmittel in einen kalten Vorformling eingeleitet wird und hier kondensiert. Problematisch ist hier die Sicherstellung einer vollständigen Kondensatbildung auf der gesamten Innenfläche des Vorformlings, da das einströmende heiße Sterilisationsmittel die Innenwandtemperatur des Vorformlings erhöht. Darüber hinaus tritt auch hier das Sterilisationsmittel nach seiner Verdampfung im Bereich der Heizung unkontrolliert innerhalb der Heizung aus dem Vorformling aus.

In der EP-A 2 138 298 wird eine Vorrichtung beschrieben, bei der vorsorglich sowohl vor dem verwendeten Blasmodul als auch hinter dem verwendeten Blasmodul Sterilisiereinrichtungen angeordnet sind. Hieraus resultiert ein sehr großer maschinenbaulicher Aufwand.

In der WO 2010/020530 A1 wird die Anordnung einer Sterilisiereinrichtung zwischen einer Heizung und dem Blasmodul beschrieben. Bei diesem Verfahren ist die Eintragsmenge von Sterilisationsmittel in den Bereich des Blasmoduls nur schwer vorhersehbar. Darüber hinaus ist die Austrittsmenge an Sterilisationsmittel in die Umgebung nicht kontrollierbar und eine entsprechende Kontamination nicht ausgeschlossen.

Die DE 10 2007 050 582 A1 zeigt einen Elektronenstrahler als Sterilisiereinrichtung, der zusammen mit den zu sterilisierenden Vorformlingen bewegt wird und Elektronenstrahlung auf die Vorformlinge abgibt.

Die nachveröffentlichte EP 2 295 324 A1 zeigt einen mit den Vorformlingen umlaufenden Applikator für ein chemisches Sterilisiermittel, der von oben und in Achsrichtung des Vorformlings in die offene Vorformlingsmündung ein Sterilisieragens einsprüht.

Die EP 1 941 913 A1 zeigt eine stationäre Sterilisiereinrichtung mit mehreren oberhalb der durch die Einrichtung geführten Vorformlinge angeordneten Applikatoren, aus denen heraus ein Sterilisiermittel in die Vorformlinge eingesprüht wird.

Aufgabe der vorliegenden Erfindung ist es, ein Verfahren der einleitend genannten Art derart zu verbessern, daß in einfacher Weise eine zuverlässige Sterilisation durchgeführt werden kann.

Diese Aufgabe wird erfindungsgemäß dadurch gelöst, daß das Sterilisationsmittel wenigstens zum Teil von mindestens einer Düse in Richtung auf den Vorformling abgegeben wird, wobei eine mittlere Austrittsrichtung des Sterilisationsmittels aus der Düse heraus mit einem Neigungswinkel zu einer Längsachse des Vorformlings versehen wird.

Weitere Aufgabe der vorliegenden Erfindung ist es, eine Vorrichtung der einleitend genannten Art derart zu konstruieren, daß eine effektive Sterilisation mit geringem Aufwand durchführbar ist.

Diese Aufgabe wird erfindungsgemäß dadurch gelöst, daß mindestens eine Düse zur Abgabe des Sterilisationsmittels eine mittlere Austrittsrichtung des Sterilisationsmittels derart aufweist, daß diese mittlere Austrittsrichtung einen Neigungswinkel zu einer Längsachse des Vorformlings aufweist. Ein einfacher konstruktiver Aufbau wird dadurch unterstützt, daß die Vorformlinge an einer stationär angeordneten Sterilisiereinrichtung vorbeigefördert werden.

Durch die Neigung der mittleren Austrittsrichtung des Sterilisationsmittels relativ zur Längsachse des Vorformlings wird die Ausbildung einer turbulenten Strömung innerhalb des Vorformlings sowie ein intensiver Kontakt zwischen dem Sterilisationsmittel und der inneren Oberfläche des Vorformlings unterstützt. Der Bezug auf die mittlere Austrittsrichtung des Strömungsmittels berücksichtigt, daß typischerweise das Strömungsmittel nicht laminar aus der Düse austritt, sondern daß ausgehend von der Düse eine Aufweitung der Strömung erfolgt. Typischerweise erfolgt diese Aufweitung zumindest in einer Nähe der Düse kegelartig.

Schließlich besteht eine Aufgabe der vorliegenden Erfindung darin, eine Vorrichtung zur Blasformung von Behältern der einleitend genannten Art derart zu konstruieren, daß die Herstellung von sterilen Behältern unterstützt wird.

Diese Aufgabe wird erfindungsgemäß dadurch gelöst, daß mindestens eine Düse zur Abgabe des Sterilisationsmittels eine mittlere Austrittsrichtung des Sterilisationsmittels derart aufweist, daß diese mittlere Austrittsrichtung einen Neigungswinkel zu einer Längsachse des Vorformlings aufweist. Auch hierbei wird ein einfacher konstruktiver Aufbau dadurch unterstützt, daß die Vorformlinge an einer stationär angeordneten Sterilisiereinrichtung vorbeigefördert werden, die Vorrichtung also eine stationär angeordnete Sterilisiereinrichtung und Mittel zum Vorbeifördern der Vorformlinge an der Sterilisiereinrichtung aufweist.

Eine vorteilhafte Beeinflussung der Strömung erfolgt dadurch, daß der Neigungswinkel im Bereich von 10° bis 80° liegt.

Insbesondere ist daran gedacht, daß der Neigungswinkel im Intervall von 20° bis 60° liegt.

Als besonders vorteilhaft erweist es sich, daß der Neigungswinkel im Bereich von 30° bis 35° liegt.

Ein intensiver Kontakt des Sterilisationsmittels mit der Innenwandung des Vorformlings wird dadurch unterstützt, daß das Sterilisationsmittel aus der Düse heraus und in einen Mündungsabschnitt des Vorformlings hineinströmt.

Ebenfalls trägt es zu einer vorteilhaften Strömungsführung bei, daß eine Austrittsöffnung der Düse dichter an einem Rand des Mündungsabschnittes als relativ zur Längsachse positioniert wird.

Kontinuierliche Bewegungsabläufe während der Durchführung der Sterilisierung werden dadurch unterstützt, daß ein Austrittsbereich der Düse mit einem Abstand zu einem Rand des Mündungsabschnittes positioniert wird.

Zur Erreichung einer Aktivierung des Sterilisationsmittels wird vorgeschlagen, daß die Temperatur des vorformlings im Bereich einer inneren Oberfläche während der Durchführung der Sterilisierung mindestens 80°C beträgt.

Als besonders vorteilhaft für eine Aktivierung erweist es sich, daß die Temperatur in einem Bereich von 100°C bis 130°C liegt.

In den Zeichnungen sind Ausführungsbeispiele der Erfindung schematisch dargestellt. Es zeigen:
- Fig. 1: Eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur Veranschaulichung eines grundsätzlichen Aufbaus einer Vorrichtung zur Blas-formung von Behältern,
- Fig. 4: eine modifizierte Heizstrecke mit vergrößerter Heizkapazität,
- Fig. 5: eine schematische Darstellung eines Heizmoduls einer Blasmaschine, bei der im Bereich des Heizmoduls eine Sterilisiereinrichtung angeordnet ist,
- Fig. 6: eine gegenüber Fig. 5 abgewandelte Ausführungsform und
- Fig. 7: eine schematische Darstellung eines Längsschnittes durch einen Vorformling mit zugeordneter Düse zur Abgabe eines Sterilisationsmittels.

Vor einer Erläuterung des Detailaufbaus der Vorrichtung zum Sterilisieren der Vorformlinge (1) durch Anwendung eines Sterilisationsmittels sowie vor einer Erläuterung eines konkreten Einbaues einer entsprechenden Vorrichtung in eine Blasmaschine soll nachfolgend zunächst der grundsätzliche Aufbau einer Blasmaschine beschrieben werden.

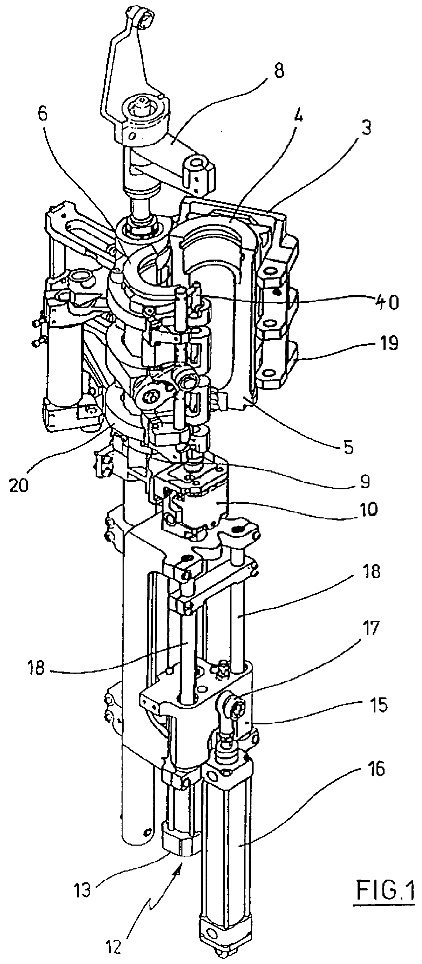

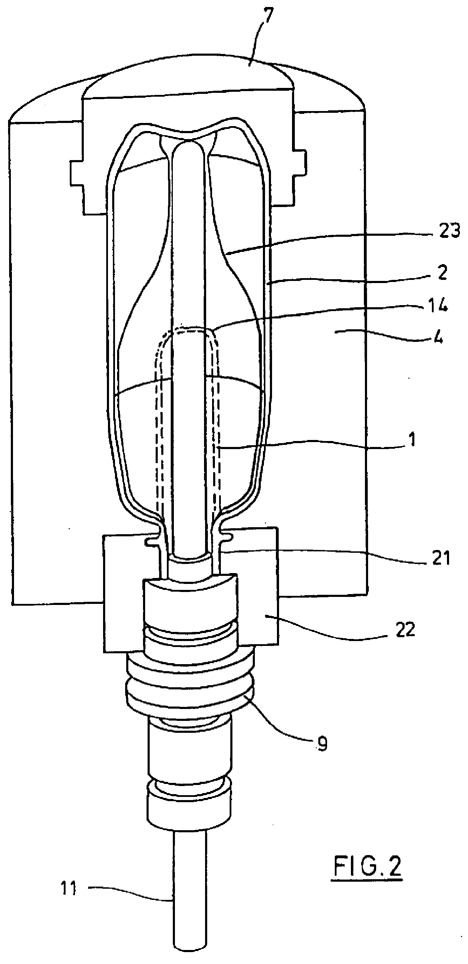

Der prinzipielle Aufbau einer Vorrichtung zur Umformung von Vorformlingen (1) in Behälter (2) ist in Fig. 1 und in Fig. 2 dargestellt.

Die Vorrichtung zur Formung des Behälters (2) besteht im wesentlichen aus einer Blasstation (3), die mit einer Blasform (4) versehen ist, in die ein vorformling (1) einsetzbar ist. Der Vorformling (1) kann ein spritzgegossenes Teil aus Polyethylenterephthalat sein. Zur Ermöglichung eines Einsetzens des Vorformlings (1) in die Blasform (4) und zur Ermöglichung eines Herausnehmens des fertigen Behälters (2) besteht die Blasform (4) aus Formhälften (5, 6) und einem Bodenteil (7), das von einer Hubvorrichtung (8) positionierbar ist. Der Vorformling (1) kann im Bereich der Blasstation (3) von einem Transportdorn (9) gehalten sein, der gemeinsam mit dem Vorformling (1) eine Mehrzahl von Behandlungsstationen innerhalb der Vorrichtung durchläuft. Es ist aber auch möglich, den Vorformling (1) beispielsweise über Zangen oder andere Handhabungsmittel direkt in die Blasform (4) einzusetzen.

Zur Ermöglichung einer Druckluftzuleitung ist unterhalb des Transportdornes (9) ein Anschlußkolben (10) angeordnet, der dem Vorformling (1) Druckluft zuführt und gleichzeitig eine Abdichtung relativ zum Transportdorn (9) vornimmt. Bei einer abgewandelten Konstruktion ist es grundsätzlich aber auch denkbar, feste Druckluftzuleitungen zu verwenden.

Eine Reckung des vorformlings (1) erfolgt mit Hilfe einer Reckstange (11), die von einem Zylinder (12) positioniert wird. Grundsätzlich ist es aber auch denkbar, eine mechanische Positionierung der Reckstange (11) über Kurvensegmente durchzuführen, die von Abgriffrollen beaufschlagt sind. Die Verwendung von Kurvensegmenten ist insbesondere dann zweckmäßig, wenn eine Mehrzahl von Blasstationen (3) auf einem rotierenden Blasrad angeordnet sind. Eine Verwendung von Zylindern (12) ist zweckmäßig, wenn ortsfest angeordnete Blasstationen (3) vorgesehen sind.

Bei der in Fig. 1 dargestellten Ausführungsform ist das Recksystem derart ausgebildet, daß eine Tandem-Anordnung von zwei Zylindern (12) bereitgestellt ist. Von einem Primärzylinder (13) wird die Reckstange (11) zunächst vor Beginn des eigentlichen Reckvorganges bis in den Bereich eines Bodens (14) des Vorformlings (1) gefahren. während des eigentlichen Reckvorganges wird der Primärzylinder (13) mit ausgefahrener Reckstange gemeinsam mit einem den Primärzylinder (13) tragenden Schlitten (15) von einem Sekundärzylinder (16) oder über eine Kurvensteuerung positioniert. Insbesondere ist daran gedacht, den Sekundärzylinder (16) derart kurvengesteuert einzusetzen, daß von einer Führungsrolle (17), die während der Durchführung des Reckvorganges an einer Kurvenbahn entlang gleitet, eine aktuelle Reckposition vorgegeben wird. Die Führungsrolle (17) wird vom Sekundärzylinder (16) gegen die Führungsbahn gedrückt. Der Schlitten (15) gleitet entlang von zwei Führungselementen (18).

Nach einem Schließen der im Bereich von Trägern (19, 20) angeordneten Formhälften (5, 6) erfolgt eine Verriegelung der Träger (19, 20) relativ zueinander mit Hilfe einer Verriegelungseinrichtung (40).

zur Anpassung an unterschiedliche Formen eines Mündungsabschnittes (21) des Vorformlings (1) ist gemäß Fig. 2 die Verwendung separater Gewindeeinsätze (22) im Bereich der Blasform (4) vorgesehen.

Fig. 2 zeigt zusätzlich zum geblasenen Behälter (2) auch gestrichelt eingezeichnet den Vorformling (1) und schematisch eine sich entwickelnde Behälterblase (23).

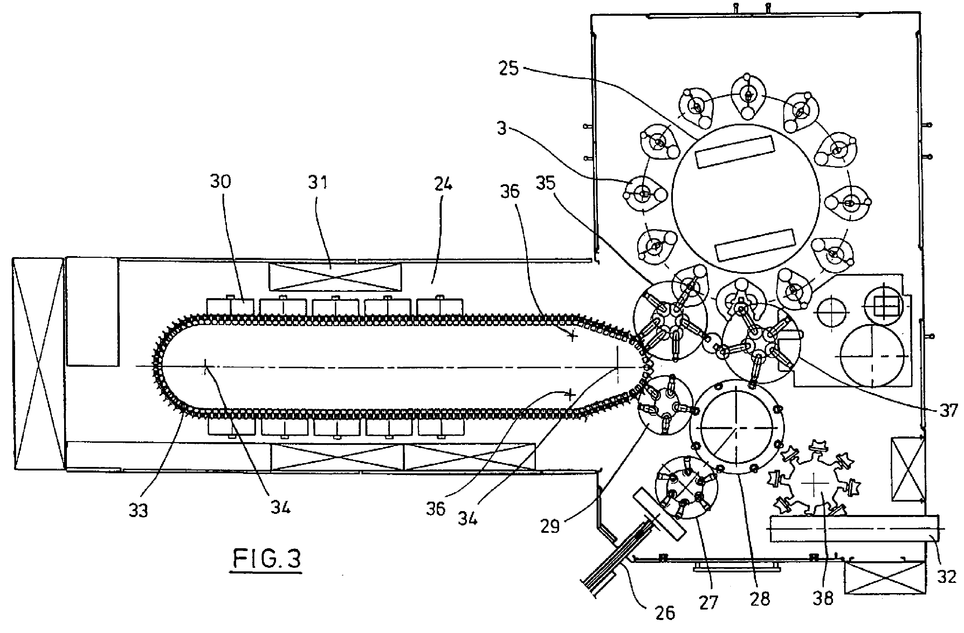

Fig. 3 zeigt den grundsätzlichen Aufbau einer Blasmaschine, die mit einer Heizstrecke (24) sowie einem rotierenden Blasrad (25) versehen ist. Ausgehend von einer Vorformlingseingabe (26) werden die Vorformlinge (1) von Übergaberädern (27, 28, 29) in den Bereich der Heizstrecke (24) transportiert. Entlang der Heizstrecke (24) sind Heizstrahler (30) sowie Gebläse (31) angeordnet, um die Vorformlinge (1) zu temperieren. Nach einer ausreichenden Temperierung der Vorformlinge (1) werden diese an das Blasrad (25) übergeben, in dessen Bereich die Blasstationen (3) angeordnet sind. Die fertig geblasenen Behälter (2) werden von weiteren Übergaberädern einer Ausgabestrecke (32) zugeführt.

Um einen Vorformling (1) derart in einen Behälter (2) umformen zu können, daß der Behälter (2) Materialeigenschaften aufweist, die eine lange Verwendungsfähigkeit von innerhalb des Behälters (2) abgefüllten Lebensmitteln, insbesondere von Getränken, gewährleisten, müssen spezielle Verfahrensschritte bei der Beheizung und Orientierung der Vorformlinge (1) eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind bei-spielsweise PET, PEN oder PP.

Die Expansion des Vorformlings (1) während des Orientierungsvorganges erfolgt durch Druckluftzuführung. Die Druckluftzuführung ist in eine Vorblasphase, in der Gas, zum Beispiel Preßluft, mit einem niedrigen Druckniveau zugeführt wird und in eine sich anschließende Hauptblasphase unterteilt, in der Gas mit einem höheren Druckniveau zugeführt wird. Während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, daß bei der dargestellten Ausführungsform die Heizstrecke (24) aus einer Vielzahl umlaufender Transportelemente (33) ausgebildet ist, die kettenartig aneinandergereiht und entlang von Umlenkrädern (34) geführt sind. Insbesondere ist daran gedacht, durch die kettenartige Anordnung eine im wesentlichen rechteckförmige Grundkontur aufzuspannen. Bei der dargestellten Ausführungsform werden im Bereich der dem Übergaberad (29) und einem Eingaberad (35) zugewandten Ausdehnung der Heizstrecke (24) ein einzelnes relativ groß dimensioniertes Umlenkrad (34) und im Bereich von benachbarten Umlenkungen zwei vergleichsweise kleiner dimensionierte Umlenkräder (36) verwendet. Grundsätzlich sind aber auch beliebige andere Führungen denkbar.

Zur Ermöglichung einer möglichst dichten Anordnung des Übergaberades (29) und des Eingaberades (35) relativ zueinander erweist sich die dargestellte Anordnung als besonders zweckmäßig, da im Bereich der entsprechenden Ausdehnung der Heizstrecke (24) drei Umlenkräder (34, 36) positioniert sind, und zwar jeweils die kleineren Umlenkräder (36) im Bereich der Überleitung zu den linearen Verläufen der Heizstrecke (24) und das größere Umlenkrad (34) im unmittelbaren Übergabebereich zum Übergaberad (29) und zum Eingaberad (35). Alternativ zur Verwendung von kettenartigen Transportelementen (33) ist es beispielsweise auch möglich, ein rotierendes Heizrad zu verwenden.

Nach einem fertigen Blasen der Behälter (2) werden diese von einem Entnahmerad (37) aus dem Bereich der Blasstationen (3) herausgeführt und über das Übergaberad (28) und ein Ausgaberad (38) zur Ausgabestrecke (32) transportiert.

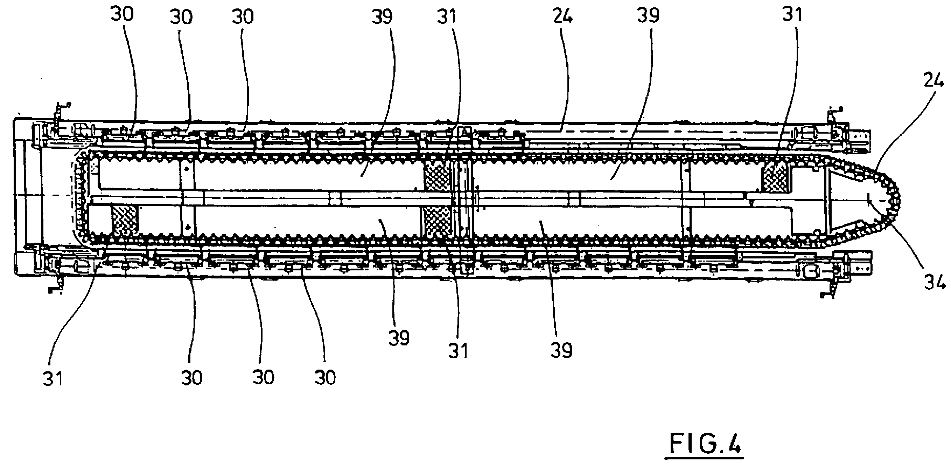

In der in Fig. 4 dargestellten modifizierten Heizstrecke (24) können durch die größere Anzahl von Heizstrahlern (30) eine größere Menge von Vorformlingen (1) je Zeiteinheit temperiert werden. Die Gebläse (31) leiten hier Kühlluft in den Bereich von Kühlluftkanälen (39) ein, die den zugeordneten Heizstrahlern (30) jeweils gegenüberliegen und über Ausströmöffnungen die Kühlluft abgeben. Durch die Anordnung der Ausströmrichtungen wird eine Strömungsrichtung für die Kühlluft im wesentlichen quer zu einer Transportrichtung der Vorformlinge (1) realisiert. Die Kühlluftkanäle (39) können im Bereich von den Heizstrahlern (30) gegenüberliegenden Oberflächen Reflektoren für die Heizstrahlung bereitstellen, ebenfalls ist es möglich, über die abgegebene Kühlluft auch eine Kühlung der Heizstrahler (30) zu realisieren.

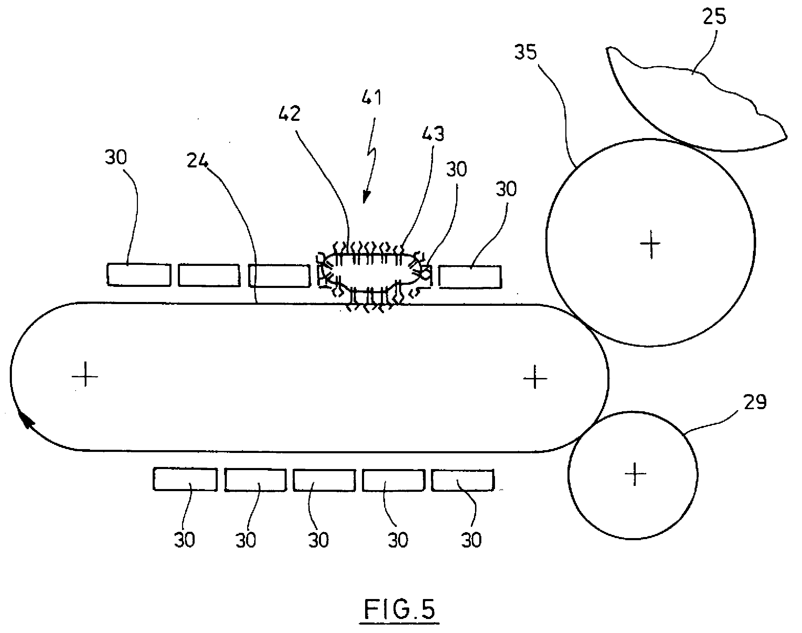

Fig. 5 zeigt schematisch und stark vereinfacht eine Anordnung ähnlich zur Darstellung in Fig. 3 mit zusätzlicher Anordnung einer Sterilisiereinrichtung (41), hier beispielhaft im Bereich der Heizstrecke (24). Gemäß dem in Fig. 5 dargestellten Ausführungsbeispiel ist im Bereich der Sterilisiereinrichtung (41) eine umlaufend angetriebene Transporteinrichtung (42) für Halteelemente (43) angeordnet, die eine Fixierung und/oder Positionierung der vorformlinge (1) mindestens während eines Teiles der Zeitdauer des Sterilisationsvorganges ermöglichen.

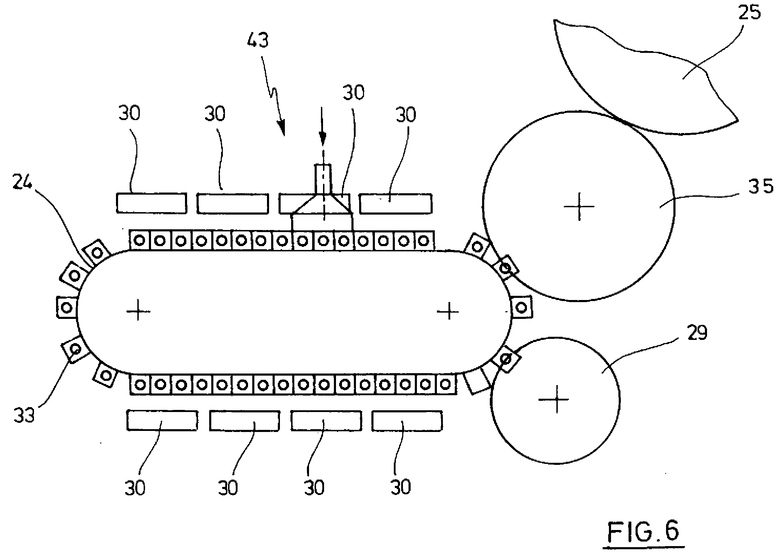

Fig. 6 zeigt eine abgewandelte Ausführungsform, bei der im Bereich der Sterilisiereinrichtung (41) keine zusätzlichen Halteelemente (42) verwendet werden. Zusätzlich eingezeichnet sind die Transportelemente (33) der Heizstrecke (24).

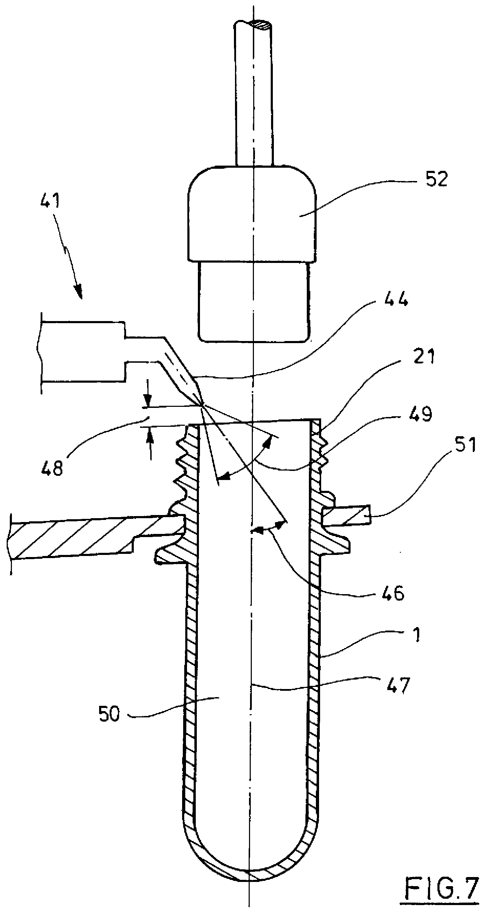

Gemäß der Ausführungsform in Fig. 7 weist die Sterilisiereinrichtung (41) eine Düse (44) zur Abgabe des Sterilisationsmittels auf. Die Düse (44) definiert eine mittlere Austrittsrichtung (45) des Sterilisationsmittels. Die Austrittsrichtung (45) weist einen Neigungswinkel (46) zu einer Längsachse (47) des Vorformlings (1) auf. Bei der dargestellten Anordnung des Vorformlings (1) mit einer in einer vertikalen Richtung orientierten Längsachse (47) entspricht der Neigungswinkel (46) auch dem Winkel zwischen der Austrittsrichtung (45) und der vertikalen Richtung.

Während der in Fig. 7 dargestellten Durchführung des Sterilisationsvorganges ist insbesondere daran gedacht, die Düse (44) ortsfest anzuordnen und den Vorformling (1) an der Düse (44) vorbeizubewegen. Besonders zweckmäßig ist es, wenn beim dargestellten Ausführungsbeispiel mit einer Orientierung der Vorformlinge (1) mit ihrem Mündungsabschnitt (21) nach oben die Düse (44) mit einem Abstand (48) leicht oberhalb des Mündungsabschnittes (21) positioniert ist. Das aus der Düse (44) austretende Sterilisationsmittel breitet sich typischerweise ausgehend von der Düse (44) mit einem Expansionswinkel (49) aus.

Grundsätzlich ist es ebenfalls möglich, die Düse in einen Innenraum (50) des Vorformlings (1) einzuführen oder den Austrittsbereich der Düse (44) bündig mit einem Rand des Vorformlings (1) anzuordnen. Bei einer Anordnung des Vorformlings (1) mit seinem Mündungsabschnitt (21) in lotrechter Richtung nach unten kann die Anordnung gemäß Fig. 7 um 180° gedreht werden.

Beim dargestellten Ausführungsbeispiel wird der Vorformling (1) während der Dauer des Sterilisationsvorganges von einem Halteelement (51) positioniert. Das Halteelement (51) kann zangenartig ausgebildet sein. Vor bzw. nach der Durchführung des Sterilisationsvorganges kann der Vorformling (1) auch von einem Halteelement (52) positioniert werden, das in den Mündungsabschnitt (21) eingeführt wird oder den Mündungsabschnitt (21) glockenartig umgibt. Das Halteelement (52) wird dabei vorzugsweise am Mündungsabschnitt (21) festgeklemmt. In Richtung der Längsachse (47) ist das Halteelement (52) vorzugsweise höhenpositionierbar angeordnet.

Das Sterilisationsmittel wird vorzugsweise in einem gasförmigen Zustand in den Innenraum (48) eingeleitet. Insbesondere ist an eine Temperatur des Sterilisationsmittels von oberhalb 100°C gedacht. Vorzugsweise weist der Vorformling (1) bei der Durchführung des Sterilisationsvorganges im Bereich seiner zu sterilisierenden inneren Oberfläche eine Temperatur von oberhalb 80°C auf. Hinsichtlich des Sterilisationsmittels ist insbesondere an die Verwendung von Wasserstoffperoxid gedacht.

Gemäß typischen Prozeßbedingungen weist das Sterilisationsmittel während der Durchführung der Sterilisierung eine Temperatur im Bereich von 100°C bis 130°C auf. Der Vorformling (1) weist während der Durchführung der Sterilisierung mindestens im Bereich seiner inneren Oberfläche eine Temperatur von 100°C bis 130°C auf. Eine typische Sterilisationsdauer beträgt etwa 0,1 bis 0,5 sec. Als Sterilisationsmittel wird bevorzugt verdampftes Wasserstoffperoxid verwendet, das mit Heißluft gemischt wird. Die Wasserstoffperoxidkonzentration beträgt dabei etwa 15 bis 35 Gewichtsprozent.

Gemäß dem Ausführungsbeispiel in Fig. 7 weist die Düse (44) einen Innendurchmesser von 3,9 Millimeter und bezüglich der Austrittsrichtung (45) eine Erstreckung von 56 Millimeter auf. Der Abstand (48) beträgt beim dargestellten Ausführungsbeispiel 10 Millimeter.

Das Sterilisationsmittel wird typischerweise bei einer Verdampfertemperatur von 150°C in den gasförmigen Zustand überführt. Die vorstehend bereits erwähnte Konzentration an Wasserstoffperoxid entspricht einem Gehalt von etwa 0,05 bis 0,5 Kilogramm H₂O₂/Nm³.

Bei einer ortsfesten Anordnung der Sterilisiereinrichtung (41) und einer Vorbeiführung der Vorformlinge (1) an der Sterilisiereinrichtung (41) ist insbesondere daran gedacht, kontinuierlich das Sterilisationsmittel abzugeben. Überschüssiges Sterilisationsmittel oder wieder aus dem Vorformling (1) austretendes Sterilisationsmittel kann abgesaugt werden, um eine unkontrollierte Ausbreitung zu vermeiden.

## Patentansprüche

1. Verfahren zum Sterilisieren von Vorformlingen aus einem thermoplastischen Material, die zur Herstellung von blasgeformten Behältern vorgesehen sind sowie bei dem ein Sterilisationsmittel in den Bereich des Vorformlings zugeführt wird, sowie bei dem die Vorformlinge vor ihrer Blasverformung erwärmt werden, **dadurch gekennzeichnet, daß** das Sterilisationsmittel wenigstens zum Teil von mindestens einer Düse (44) in Richtung auf den Vorformling (1) abgegeben wird, wobei eine mittlere Austrittsrichtung (45) des Sterilisationsmittel aus der Düse (44) heraus mit einem Neigungswinkel (46) zu einer Längsachse (47) des Vorformlings (1) versehen wird, wobei die Vorformlinge (1) an einer stationär angeordneten Sterilisiereinrichtung (41) vorbeigefördert werden.

2. Verfahren nach Anspruch 1, **dadurch gekennzeichnet, daß** die Temperatur des Vorformlings (1) im Bereich einer inneren Oberfläche während der Durchführung der Sterilisierung mindestens 80°C beträgt.

3. Verfahren nach Anspruch 2, **dadurch gekennzeichnet, daß** die Temperatur in einem Bereich von 100°C bis 130°C liegt.

4. Verfahren nach einem der Ansprüche 1 bis 3, **dadurch gekennzeichnet, daß** der Neigungswinkel (46) im Bereich von 10° bis 80° liegt.

5. Verfahren nach einem der Ansprüche 1 bis 4, **dadurch gekennzeichnet, daß** der Neigungswinkel im Intervall von 20° bis 60° liegt.

6. Verfahren nach einem der Ansprüche 1 bis 5, **dadurch gekennzeichnet, daß** der Neigungswinkel (46) im Bereich von 30° bis 35° liegt.

7. Verfahren nach einem der Ansprüche 1 bis 6, **dadurch gekennzeichnet, daß** das Sterilisationsmittel aus der Düse (44) heraus und in einen Mündungsabschnitt (21) des Vorformlings (1) hineinströmt.

8. Verfahren nach einem der Ansprüche 1 bis 7, **dadurch gekennzeichnet, daß** eine Austrittsöffnung der Düse (44) dichter an einem Rand des Mündungsabschnittes (21) als relativ zur Längsachse (47) positioniert wird.

9. Verfahren nach einem der Ansprüche 1 bis 8, **dadurch gekennzeichnet, daß** ein Austrittsbereich der Düse (44) mit einem Abstand (48) zu einem Rand des Mündungsabschnittes (21) positioniert wird.

10. Vorrichtung zum Sterilisieren von Vorformlingen aus einem thermoplastischen Material, die zur Herstellung von blasgeformten Behältern vorgesehen sind, die eine Zuführeinrichtung zur Beaufschlagung mindestens eines Teiles des Vorformlings mit einem Sterilisationsmittel aufweist, mit einer stationär angeordneten Sterilisiereinrichtung (41), **dadurch gekennzeichnet, daß** mindestens eine Düse (44) zur Abgabe des Sterilisationsmittels eine mittlere Austrittsrichtung (45) des Sterilisationsmittels derart aufweist, daß diese mittlere Austrittsrichtung (45) einen Neigungswinkel (46) zu einer Längsachse (47) des Vorformlings (1) aufweist, wobei Mittel vorgesehen sind, um die Vorformlinge (1) an der Sterilisiereinrichtung (41) vorbeizufördern.

11. Vorrichtung nach Anspruch 10, **dadurch gekennzeichnet, daß** der Neigungswinkel (46) im Intervall von 10° bis 80° liegt.

12. Vorrichtung nach Anspruch 10 oder 11, **dadurch gekennzeichnet, daß** eine Austrittsöffnung der Düse (44) dichter an einem Rand des Mündungsabschnittes (21) als relativ zur Längsachse (47) positioniert wird.

13. Vorrichtung nach einem der Ansprüche 10 bis 12, **dadurch gekennzeichnet, daß** ein Austrittsbereich der Düse (44) mit einem Abstand (48) zu einem Rand des Mündungsabschnittes (21) positioniert wird.

## Claims

1. Method for sterilising preforms that are made of a thermoplastic material and intended for the production of blow-moulded containers, in which a sterilising agent is introduced into the area of the preform, and the preforms are heated prior to being blow-moulded, **characterised in that** at least some of the sterilising agent is dispensed by at least one nozzle (44) in the direction of the preform (1), a mean discharge direction (45) of the sterilising agent from the nozzle (44) being provided with an angle of inclination (46) with respect to a longitudinal axis (47) of the preform (1), and the preforms (1) being moved past a stationary sterilising device (41).

2. Method according to Claim 1, **characterised in that** the temperature of the preform (1) in the area of an inner surface is at least 80°C while the sterilisation is being carried out.

3. Method according to Claim 2, **characterised in that** the temperature is in a range from 100°C to 130°C.

4. Method according to any one of Claims 1 to 3, **characterised in that** the angle of inclination (46) is in the range from 10° to 80°.

5. Method according to any one of Claims 1 to 4, **characterised in that** the angle of inclination (46) is the interval from 20° to 60°.

6. Method according to any one of Claims 1 to 5, **characterised in that** the angle of inclination (46) is in the range from 30° to 35°.

7. Method according to any one of Claims 1 to 6, **characterised in that** the sterilising agent flows out of the nozzle (44) and into a mouth section (21) of the preform (1).

8. Method according to any one of Claims 1 to 7, **characterised in that** a discharge opening of the nozzle (44) is positioned closer to an edge of the mouth section (21) than relative to the longitudinal axis (47).

9. Method according to any one of Claims 1 to 8, **characterised in that** a discharge area of the nozzle (44) is positioned at a distance (48) from an edge of the mouth section (21).

10. Device for sterilising preforms that are made of a thermoplastic material and intended for the production of blow-moulded containers, comprising a feed device for supplying a sterilising agent to at least a portion of the preform, including a stationary sterilization device (41), **characterised in that** at least one nozzle (44) for dispensing the sterilising agent has a mean discharge direction (45) of the sterilising agent in such a way that this mean discharge direction (45) has an angle of inclination (46) with respect to a longitudinal axis (47) of the preform, means being provided for moving the preforms (1) past the sterilising device (41).

11. Device according to Claim 10, **characterised in that** the angle of inclination (46) is in the interval from 10° to 80°.

12. Device according to Claim 10 or 11, **characterised in that** a discharge opening of the nozzle (44) is positioned closer to an edge of the mouth section (21) than relative to the longitudinal axis (47).

13. Device according to any one of Claims 10 to 12, **characterised in that** a discharge area of the nozzle (44) is positioned at a distance (48) from an edge of the mouth section (21).

## Revendications

1. Procédé de stérilisation de préformes en un matériau thermoplastique destinées à la fabrication de récipients moulés par soufflage, dans le cadre duquel un agent de stérilisation est introduit dans la zone de la préforme, et dans le cadre duquel les préformes sont réchauffées avant leur moulage par soufflage, **caractérisé en ce que** l'agent de stérilisation est, en partie au moins, éjecté par au moins une buse (44) en direction de la préforme (1), une direction moyenne (45) d'éjection de l'agent de stérilisation hors de la buse (44) formant un angle d'inclinaison (46) par rapport à un axe longitudinal (47) de la préforme (1) et les préformes (1) étant transportées de façon à passer devant un dispositif de stérilisation (41) d'agencement stationnaire.

2. Procédé selon la revendication 1, **caractérisé en ce que** la température de la préforme (1) au niveau d'une surface intérieure pendant l'opération de stérilisation est d'au moins 80 °C.

3. Procédé selon la revendication 2, **caractérisé en ce que** la température est comprise dans un intervalle de 100 °C à 130 °C.

4. Procédé selon l'une des revendications 1 à 3, **caractérisé en ce que** l'angle d'inclinaison (46) est compris dans un intervalle de 10° à 80°.

5. Procédé selon l'une des revendications 1 à 4, **caractérisé en ce que** l'angle d'inclinaison est compris dans un intervalle de 20° à 60°.

6. Procédé selon l'une des revendications 1 à 5, **caractérisé en ce que** l'angle d'inclinaison (46) est compris dans un intervalle de 30° à 35°.

7. Procédé selon l'une des revendications 1 à 6, **caractérisé en ce que** l'agent de stérilisation est éjecté de la buse (44) pour pénétrer dans une section d'embouchure (21) de la préforme (1).

8. Procédé selon l'une des revendications 1 à 7, **caractérisé en ce qu'**un orifice d'éjection de la buse (44) est positionné en un point relativement plus proche d'un bord de la section d'embouchure (21) que de l'axe longitudinal (47).

9. Procédé selon l'une des revendications 1 à 8, **caractérisé en ce qu'**une zone d'éjection de la buse (44) est positionnée à une certaine distance (48) d'un bord de la section d'embouchure (21).

10. Dispositif de stérilisation de préformes en un matériau thermoplastique destinées à la fabrication de récipients moulés par soufflage, doté d'un dispositif d'éjection d' un agent de stérilisation agissant sur une partie au moins de la préforme, avec un dispositif de stérilisation stationnaire (41), **caractérisé en ce qu'**au moins une buse (44) d'éjection de l'agent de stérilisation présente une direction moyenne (45) d'éjection de l'agent de stérilisation telle que celle-ci forme un angle d'inclinaison (46) par rapport à un axe longitudinal (47) de la préforme (1), des moyens étant prévus pour assurer le transport des préformes (1) de façon qu'elles passent devant le dispositif de stérilisation (41).

11. Dispositif selon la revendication 10, **caractérisé en ce que** l'angle d'inclinaison (46) est compris dans un intervalle de 10° à 80°.

12. Dispositif selon la revendication 10 ou 11, **caractérisé en ce que** un orifice d'éjection de la buse (44) est positionné en un point relativement plus proche d'un bord de la section d'embouchure (21) que de l'axe longitudinal (47).

13. Dispositif selon l'une des revendications 10 à 12, **caractérisé en ce que** une zone d'éjection de la buse (44) est positionnée à une certaine distance (48) d'un bord de la section d'embouchure (21).
